# EUROPEAN PATENT SPECIFICATION

(11) **EP 3 775 914 B1**
(45) Date of publication and mention of the grant of the patent: **20.09.2023**
(21) Application number: 19712804.4
(22) Date of filing: 29.03.2019
(51) Int. Cl.: G01N 33/68

(54) **METHOD OF DIAGNOSING HEART MUSCLE DAMAGE**
METHOD ZUR DIAGNOSE VON HERZMUSKELSCHÄDEN
PROCÉDÉ DE DIAGNOSTIC DE L'ENDOMMAGEMENT DU MUSCLE CARDIAQUE

(30) Priority: 29.03.2018 EP 18164958
(43) Date of publication of application: 17.02.2021
(73) Proprietor: Lange, Rüdiger, 82335 Berg (DE); Krane, Markus, 80637 München (DE)
(72) Inventor: DOPPLER, Stefanie, 80636 München (DE); LAHM, Harald, 81375 München (DE); DREßEN, Martina, 85221 Dachau (DE); LANGE, Rüdiger, 82335 Berg (DE); KRANE, Markus, 80637 München (DE)
(74) Representative: Maiwald GmbH
(86) International application number: PCT/EP2019/058014
(87) International publication number: WO 2019/185869

(56) References cited:
- JP-A- 2017 009 329
- DAVID Y. BAREFIELD ET AL: "Experimental Modeling Supports a Role for MyBP-HL as a Novel Myofilament Component in Arrhythmia and Dilated Cardiomyopathy", CIRCULATION, vol. 136, no. 16, 4 August 2017 (2017-08-04), pages 1477-1491, XP055473449, ISSN: 0009-7322, DOI: 10.1161/CIRCULATIONAHA.117.028585
- JULIA ASP ET AL: "Comparison of human cardiac gene expression profiles in paired samples of right atrium and left ventricle collected in vivo", PHYSIOLOGICAL GENOMICS, vol. 44, no. 1, 1 January 2012 (2012-01-01), pages 89-98, XP055473597, US ISSN: 1094-8341, DOI: 10.1152/physiolgenomics.00137.2011
- DEVIN W. KEHL ET AL: "Biomarkers in acute myocardial injury", TRANSLATIONAL RESEARCH, vol. 159, no. 4, 1 April 2012 (2012-04-01) , pages 252-264, XP055473623, NL ISSN: 1931-5244, DOI: 10.1016/j.trsl.2011.11.002
- KAWAI EIICHIRO ET AL: "Determination of Phase-specific Biomarkers of Viral Myocarditis Induced by Theiler's virus Using Multivariate Analyses of Viral Genome, Troponin, Transcriptome and Echocardiography Data", CIRCULATION RESEARCH, GRUNE AND STRATTON, BALTIMORE, US , vol. 113, no. 4, Suppl. S 1 August 2013 (2013-08-01), page A229, XP009505250, ISSN: 0009-7330 Retrieved from the Internet: URL:http://circres.ahajournals.org/content /113/Suppl_1/A229
- David Y Barefield ET AL: "Abstract 20693: MYBPHL is a Novel Myofilament Protein Implicated in Arrhythmia and Cardiomyopathy | Circulation", , 14 November 2017 (2017-11-14), XP055474258, Retrieved from the Internet: URL:http://circ.ahajournals.org/content/13 6/Suppl_1/A20693 [retrieved on 2018-05-11]

## Description

### Field of the invention

The present invention relates to methods of diagnosing heart muscle tissue damage, determining the extent of heart muscle tissue damage and differentiating between atrial heart muscle damage and ventricular heart muscle damage in a patient.

### Background

Heart diseases, especially coronary artery diseases (CAD), are the leading causes of morbidity and mortality in developed countries. Based on population statistics it can be calculated that approximately 310 heart attacks per hundred-thousand people older than 18 years-old and approximately 150 deaths per hundred-thousand adults can be expected each year in developed nations. In the cardiovascular medicine, enzymes and other proteins that are released from the dying heart have been identified as biomarker. Particularly, they are measurable for example after a heart attack in high concentration in blood. The first biomarkers for heart muscle damage have been described in the 1950'. Among those Aspartate-Aminotransferase (AST) and Lactate-Dehydrogenase (LDH) were described. In the 1970' and 1980', further biomarkers were discovered, e.g. Creatine Kinase (CK), its isoenzyme Creatine Kinase-Myocardial band (CK-MB), myoglobin and troponin (troponin T and troponin I). The troponins and CKMB are still in clinical use for the identification of heart muscle tissue damages. The measurement of the concentration of these biomarkers in the blood is usually periodically repeated since an increase and/or a decrease in the concentration of these biomarkers in the blood allow drawing conclusions about the occurrence or the beginning of a heart attack, the extent of the heart attack and the success of a therapy against heart attack.

All biomarkers previously cited have in common that they do not allow differentiation between atrial heart muscle damage (in the heart atrium) and ventricular heart muscle damage (in the ventricle). No specific biomarker for atrial heart muscle damage or for ventricular heart muscle damage has been discovered up to now. Such a specific biomarker would be highly relevant, particularly in the field of heart surgery in the context of postoperative care.

In patients with atrial fibrillation, a cardiac arrhythmia, an ablation of the heart atrium to treat the atrial fibrillation, is often performed during cardiac surgery. Ablation lines are set in the heart atrium following a specific scheme to prevent the transmission of electrical impulses that cause the atria to fibrillate rather than contract in a regular pattern. This ablation can be performed as endocardial cryoablation after opening of the right and left atrium or as epicardial radiofrequency ablation of the pulmonary veins as a concomitant procedure. Both, the endocardial cryoablation and the epicardial radiofrequency ablation cause damage to the heart muscle cells. Thus, the treatment of atrial fibrillation by ablation of a part of heart muscle tissue includes the destruction of heart muscle cells of the atrium and thereby causes a postoperative increase of unspecific biomarkers (CK-MB and Troponin) in the blood. Since the biomarkers used so far do not differentiate between atrial heart muscle damage and ventricular heart muscle damage, the postoperative evaluation to determine if the increase of the biomarkers is due to the damage occasioned by the ablation lines in the atrium or if it is due to an additional heart muscle damage of the ventricle, is not possible. For these reasons, patients often need to undergo further invasive diagnosis, e.g. coronary angiography by means of a heart catheter, in order, for example, to exclude a perioperative heart attack. Beside this indication, a biomarker reflecting an atrial damage could be of high interest as a parameter to monitor the success of an ablation therapy, for instance after interventional, catheter-based ablation therapy.

David Y Barefield et al. ("Experimental Modeling Supports a Role for MyBP-HL as a Novel Myofilament Component in Arrhythmia and Dilated Cardiomyopathy", CIRCULATION, vol. 136, 2017, pages 1477-1491) disclose that MYBPHL truncations may increase risk for human arrhythmias and cardiomyopathy.

Julia Asp et al. ("Comparison of human cardiac gene expression profiles in paired samples of right atrium and left ventricle collected in vivo", PHYSIOLOGICAL GENOMICS, vol. 44, 2012, pages 89 to 98) compare the global gene expression in cardiac tissue from the more accessible auricula of the right atrium to expression in tissue from the left ventricle by collecting tissue samples from five men undergoing aortic valve replacement or coronary artery bypass grafting. They disclose that genes with higher expression in the ventricle were mainly associated with contractile work of the heart and that transcription in biopsies from the auricula of the right atrium indicated a wider area of functions, including immunity and defense. As such, they conclude that biopsies from the auricula of the right atrium may be suitable for various genetic studies, but not studies directly related to muscle work.

JP 2017 009329 aims to provide biomarkers for evaluating motor functions of individuals, muscle mass, a degree of skeletal muscle cell differentiation, or degree of myotube hypertrophy by a motor function assessment marker, muscle mass assessment marker, skeletal muscle cell differentiation assessment marker, and myotube hypertrophy assessment marker, which contain myosin-binding protein H derived from a skeletal muscle sample taken from an individual, or a gene that codes for the protein.

Devin W. Kehl et al. ("Biomarkers in acute myocardial injury", TRANSLATIONAL RESEARCH, vol. 159, 2012, pages 252 to 264) relates to Acute coronary syndrome (ACS) and biomarker assays to detect evidence of myocardial necrosis for the diagnosis of ACS. It is disclosed that B-type natriuretic peptides, copeptin, ischemia-modified albumin, heart-type fatty-acid-binding protein, myeloperoxidase, C-reactive protein, choline, placental growth factor, and growth-differentiation factor-15 make up a promising group of biomarkers with potential to improve prognosis and diagnosis of ACS compared with traditional markers.

E. Kawai et al. ("Determination of Phase-specific Biomarkers of Viral Myocarditis Induced by Theiler's virus Using Multivariate Analyses of Viral Genome, Troponin, Transcriptome and Echocardiography Data", CIRCULATION REASEARCH, vol. 113, 2013, page A229) conducted multivariate analyses of viral genome, serum cardiac troponin I, echocardiography, histology, and transcriptome using microarray data of the heart tissue harvested on 4 (acute) and 7 (subacute) days post infection (dpi) and found multiple high intensity cardiac lesions using echocardiography with histological myocarditis on 7 dpi, but not 4 dpi with the data suggesting that acquired immune responses contribute to cardiomyocyte damage on 7 dpi. They disclose that the chronological order of emergence of biomarker candidates was 1) viral genome and innate immunity, 2) troponin, and 3) acquired immunity and echo and histological changes.

David Y Barefield et al. ("Abstract 20693: MYBPHL is a Novel Myofilament Protein Implicated in Arrhythmia and Cardiomyopathy", CIRCULATION, 2017) identified a premature stop in MYBPHL in human DCM and disclose that MyBP-HL is a myofilament protein and is important for normal cardiac conduction system function

Accordingly, there is demand for the implementation of an atrial biomarker for the specific detection of atrial damage. Such an atrial specific biomarker will avoid unnecessary invasive medical investigation in patients, especially in patients who have undergone a treatment by ablation.

### Summary of the Invention

The present invention and its preferred embodiments are apparent from the appendant set of claims.

In various embodiments of the method described herein, the isoforms or homologs of MYBPHL have protein sequences which have at least 70 % sequence identity with the protein sequence of MYBPHL, namely, at least 70 %, 75 %, 80 %, 85 %, 90 %, 92 %, 93 %, 94 %, 95 %, 96 %, 97 %, 98 %, 99% or 99.5 % sequence identity, preferably over the entire length and preferably with the amino acid sequences set forth in SEQ ID Nos. 3 and 6. For example, the isoforms of MYBPHL are selected from the group consisting of MYBPHL isoform1 (DNA sequence: SEQ ID NO:1, coding DNA sequence: SEQ ID NO:2, protein sequence: SEQ ID NO:3) and MYBPHL isoform 2 (DNA sequence: SEQ ID NO:4, coding DNA sequence: SEQ ID NO:5, protein sequence: SEQ ID NO:6). Generally, "isoform", as used herein, refers to a functionally similar protein that shares at least 70 % sequence identity, preferably at least 75 %, 80 %, 85 %, 90 %, 92 %, 93 %, 94 %, 95 %, 96 %, 97 %, 98 %, 99 % or 99.5 % sequence identity with the respective reference protein, preferably over the entire length. Also encompassed are homologs of all isoforms explicitly disclosed herein, in particular MYBPHL isoforms 1 and 2, that share at least 70 % sequence identity with the protein sequence of a reference isoform, namely, at least 70 %, 75 %, 80 %, 85 %, 90 %, 92 %, 93 %, 94 %, 95 %, 96 %, 97 %, 98 %, 99% or 99.5 % sequence identity, preferably over the entire length and preferably with the amino acid sequences set forth in SEQ ID Nos. 3 and 6.

In certain embodiments of the method described herein, the method comprises evaluating the extent of the atrial heart muscle tissue damage in said patient by determining the ratio between the level of the biomarker for atrial heart muscle tissue damage in the sample obtained from said patient and the level of the biomarker for atrial heart muscle tissue damage in the control individual.

In various embodiments of the method described herein, the method further comprises determining the level of a further biomarker for heart muscle tissue damage, wherein the method is suitable to differentiate between atrial muscle tissue damage and ventricular muscle tissue damage. The further biomarker for heart muscle tissue damage is a biomarker different from the biomarkers for atrial heart muscle tissue damage mentioned above and can be a general biomarker for heart muscle tissue damage selected from the group consisting of CK-MB or Troponin. Alternatively or additionally, the further biomarker for heart muscle tissue damage is for example a biomarker specific for ventricular heart muscle tissue damage, for example FHL-2 (Uniprot Database entry: Q14192, protein sequence SEQ ID NO:44), SMYD2 (Uniprot Database entry: Q9NRG4, protein sequence SEQ ID NO:45), FASTKD1 (Uniprot Database entry: Q53R41, protein sequence SEQ ID NO:46), PRR33 (Uniprot Database entry: A8MZF0, protein sequence SEQ ID NO:47), SORBS2 (Uniprot Database entry: 094875, protein sequence SEQ ID NO:48), CRISPLD1 (Uniprot Database entry: Q9H336, protein sequence SEQ ID NO:49), NPHP4 (Uniprot Database entry: 075161, protein sequence SEQ ID NO:50), ANKRD2 (Uniprot Database entry: Q9GZV1, protein sequence SEQ ID NO:51), TMEM159 (Uniprot Database entry: Q96B96, protein sequence SEQ ID NO:52), ATP7A (Uniprot Database entry: Q04656, protein sequence SEQ ID NO:53), C12ORF73 (Uniprot Database entry: Q69YU5, protein sequence SEQ ID NO:54), NAV1 (Uniprot Database entry: Q8NEY1, protein sequence SEQ ID NO:8), ATP5B (Uniprot Database entry: P06576, protein sequence SEQ ID NO:55), HSPB7 (Uniprot Database entry: Q9UBY9, protein sequence SEQ ID NO:56), TMEM88 (Uniprot Database entry: Q6PEY1, protein sequence SEQ ID NO:57), WDR62 (Uniprot Database entry: 043379, protein sequence SEQ ID NO:58), ACTN3 (Uniprot Database entry: Q08043, protein sequence SEQ ID NO:59), TRIM72 (Uniprot Database entry: Q6ZMU5, protein sequence SEQ ID NO:60), EGFLAM (Uniprot Database entry: Q63HQ2, protein sequence SEQ ID NO:61), LRRC39 (Uniprot Database entry: Q96DD0, protein sequence SEQ ID NO:62), DPYSL4 (Uniprot Database entry: 014531, protein sequence SEQ ID NO:63), PFKFB2 (Uniprot Database entry: 060825, protein sequence SEQ ID NO:64), ABCB6 (Uniprot Database entry: Q9NP58, protein sequence SEQ ID NO:65), METTL2B (Uniprot Database entry: Q6P1Q9, protein sequence SEQ ID NO:66), METTL2A (Uniprot Database entry: Q96IZ6, protein sequence SEQ ID NO:67), CARNS1 (Uniprot Database entry: A5YM72, protein sequence SEQ ID NO:68), SPTBN1 (Uniprot Database entry: Q01082, protein sequence SEQ ID NO:69) or isoforms thereof.

According to the present invention, the step of determining the level of said biomarker is performed on a sample obtained from said patient, wherein the sample is full blood, blood serum or blood plasma.

In certain embodiments of the method described herein, the patient is a human.

In various embodiments of the method described herein, determining the level of the biomarker for atrial heart muscle tissue damage and/or determining the level of the further biomarker comprises determining the protein concentration of the biomarker for atrial heart muscle tissue damage and/or determining the protein concentration of the further biomarker, for example in a sample obtained from a patient, such as a blood, serum or plasma sample.

In various embodiments of the method described herein, determining the level of the biomarker comprises determining the protein concentration of the biomarker, wherein the protein concentration is determined by an immunoassay, ELISA, mass spectrometry, chromatography, Western Blot, or gel electrophoresis, such as SDS-PAGE. Additionally, circulating nucleic acids, in particular RNA, such as mRNA, may be determined. For nucleic acid detection, various nucleic acid capture and amplification methods may be used. Detection may for example be achieved by using specific detection probes that are detectably labeled. Such techniques are widely known in the art and can be chosen by those skilled in art based on their general knowledge.

The term "antibody" is used in the broadest sense and specifically covers, for example, monoclonal antibodies, polyclonal antibodies, antibodies with polyepitopic specificity, single chain antibodies, multi-specific antibodies and fragments of antibodies. Such antibodies can be chimeric, humanized, human and synthetic. The antibodies or other detection reagents used herein are preferably specific for their intended target, i.e. discriminate in their binding affinities between the target and other non-target components. The difference in binding, such as for example binding affinity, may be at least 10-fold, preferably 100-fold or more. If a detection reagent is described as being specific for a certain isoform this means that it allows discrimination over other isoforms in that it binds the targeted isoform with higher affinity, such as 10-fold higher or more, relative to the non-targeted isoforms.

"Percent (%) sequence identity" or "Percent (%)"amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. In various embodiments, the respective disclosure with respect to sequence identities relates to percentages based on the entire length of the query and/or reference sequence, in particular the reference sequence. This means that if a sequence identity of 95 % is given for a candidate sequence said sequence has 95% sequence to the reference over the entire length of the reference.

### Brief Description of the Drawings

**Figure 1** shows the results of a gene expression analysis of the biomarker MYBPHL (Figure 1A) and the biomarker FHL2 (Figure 1B) in tissues taken from left atrium (LA), right atrium (RA), and left ventricle (LV) by means of a biopsy in the patients (Reference Example).
**Figure 2** shows the results of a gene expression analysis of the biomarkers MYBPHL, FHL2, MYBPC3 and MYBPH as well as the MYBPHL isoforms MYBPHL isoform 1 and MYBPHL isoform 2 in tissues taken from 9 left ventricle muscle tissues and 9 left atrium muscle tissues obtained by means of a biopsy in patients, wherein the gene expression analysis has been performed by qPCR (Reference Example).
**Figure 3** shows the separation of the amplification products of the biomarker MYBPHL isoform 2 in tissues taken from left atriums and from left ventricles, wherein the separation has been performed by gel electrophoresis (Reference Example).
**Figure 4** shows the separation of the amplification products of the biomarker MYBPHL isoform 2 in non-cardiac tissues, wherein the separation has been performed by gel electrophoresis (Reference Example).
**Figure 5** shows the results of the measurement of MYBPHL protein concentration in a blood plasma sample taken at periodical measurement time points over a defined period of time, wherein the plasma sample is taken from patients suffering from atrial fibrillation and undergoing an ablation.
**Figure 6** shows the results of the measurement of MYBPHL protein concentration in a blood plasma sample taken at periodical measurement time points over a defined period of time, wherein the plasma sample is taken from patients suffering from atrial fibrillation and undergoing an endocardial ablation (A) or an epicardial ablation (B).
**Figure 7** shows the fold-change in the MYBPHL protein concentration in a blood plasma according to Figure 6a.
**Figure 8** shows the results of the measurement of MYBPHL protein concentration in a blood plasma sample taken from a group of patients suffering from ST-elevation myocardial infarction, e.g. patients with a definitive damage in the ventricle. The blood samples were taken at two measurement time points, one before the surgical intervention and one 72 hours after the surgical intervention, wherein the surgical intervention was performed by means of a heart catheter.
**Figure 9A** shows the results of the measurement of CK-MB protein concentration in the blood samples obtained from the same patient group as in example 2. **Figure 9B** shows the fold-change in the CK-MB protein concentration in the blood samples according to Figure 9A.
**Figure 10** shows a correlation between the MYBPHL concentration values in a blood plasma sample of 12 patients who underwent an ablation and the CK-MB concentration values in the blood plasma sample of the same patients.
**Figure 11** shows a representation of the ratio between the fold-change of MYBPHL and the fold-change of FHL-2. On the two left columns of the representation, the results for patients suffering from ST-elevation myocardial infarction are shown, while on the six left columns of the representation, the results for patients suffering from atrial fibrillation and undergoing an ablation are shown.

### Detailed Description

The invention is based on the inventors' surprising finding that biomarkers specific for heart muscle tissue damage, preferably atrial heart muscle tissue damage, selected from the group consisting of MYBPH, MYBPHL, MYOT, ASNSD1, CHFR, NTN1, RFC5, POLI, COMP, POF1B, PLA2G2A, HDAC10, ASPG, FMOD, CA13, CACNA2D2, GNL3L, COL2A1, PDLIM4, LEPREL1, OMD, DGKZ, NT5DC2, ITLN1, NTM, PRKG2, CHGB, FAM179A, CKMT1A, CKMT1B, LTBP3, SGSM1, METTL7B, LTBP2, OGDHL, PAM, SBK3, SFRP1 and/or isoforms thereof are detectable in blood samples of patients with heart muscle tissue damage, whereas those specific biomarkers are not detected in healthy and benign control tissue and thus can serve as markers for heart muscle tissue damage, preferably for atrial heart muscle tissue damage. According to the present invention, the markers are selected from MYBPHL and isoforms thereof. Isoforms of a biomarker have protein sequences which may have at least 70 % sequence identity with the protein sequence of the biomarker, namely, 70 %, 75 %, 80 %, 85 %, 90 %, 92 %, 93 %, 94 %, 95 %, 96 %, 97 %, 98 % or 99 % sequence identity and which typically share certain aspects of functionality or even have identical functionality. In various embodiments, such isoforms correspond in length to the reference sequence, i.e. do not differ in length by more than 10%, preferably by not more than 5%, even more preferably by not more than 2% of total length. The amino acid sequences of MYBPHL isoform 1 and MYBPHL isoform 2 are set forth in SEQ ID Nos: 3 and 6, respectively. If reference is made to isoforms by indicating a percentage of sequence identity, these sequences in SEQ ID Nos: 3 and 6 are to be used as references, if not explicitly indicated otherwise. It is further understood that when reference is made herein to protein database entries, said reference is intended to cover all isoforms listed under said entry. Similarly, when reference is made herein to a specific amino acid sequence, it is understood that said reference is by way of example only, and it is intended that different isoforms of said protein that may differ in their amino acid sequence, and may, for example, be listed under the database entries referenced herein, are also encompassed in the scope of the invention.

The present invention thus relates to a method of diagnosing heart muscle tissue damage, preferably atrial heart muscle tissue damage, in a patient, wherein the method comprises:
(a) determining the level of a biomarker for atrial heart muscle tissue damage selected from the group consisting of MYBPHL and isoforms thereof in a sample obtained from the patient, wherein the sample is a full blood, blood serum or blood plasma sample, wherein determining the level of the biomarker comprises determining the protein concentration of the biomarker; and
(b) comparing the level determined in step a) with the level of the biomarker for atrial heart muscle tissue damage observed in a control individual, wherein if the level determined in step a) is increased compared to the level of the biomarker for atrial heart muscle tissue damage in the control individual, said patient is diagnosed with a heart muscle tissue damage.

The method may further comprise step (c) differentiating between atrial heart muscle damage and ventricular heart muscle damage in a patient. In some embodiments, the respective methods can also cover the medical treatment that follows the diagnosis step. For example, if a patient is diagnosed with heart muscle tissue damage, said patient may be treated by administration of a drug, further medical or even surgical intervention.

The markers disclosed herein are detected in a sample obtained from the patient, wherein the sample is a full blood, blood serum or blood plasma sample. As such a method does not require expensive equipment, the costs for the diagnosis of heart muscle tissue damage, preferably atrial heart muscle tissue damage, can be reduced. Furthermore, the new method can be carried out by any trained medical personnel and therefore does not require the patient to travel to specific screening centers. This allows more frequent medical examinations. As mentioned in the introductory part, a further advantage of this method is that patients, for example, who have undergone treatment of atrial fibrillation by ablation, do not need to undergo the currently used invasive diagnosis in order, for example, to exclude a perioperative heart attack, which is a significant advantage of the methods of the present invention.

The methods of the invention are, in various embodiments, carried out *in vitro* on a sample obtained from a patient with said sample, as described above, being full blood, blood serum or blood plasma.

The present invention relates to a method of diagnosing heart muscle tissue damage, preferably atrial heart muscle tissue damage, in a patient, wherein the method comprises determining the level of a biomarker for atrial heart muscle tissue damage selected from the group consisting of MYBPHL (Myosin-binding protein H-like; Uniprot Database entry: A2RUH7) and isoforms thereof, in particular the isoforms that are listed under the given database entries with the ones listed herein by reference to their amino acid sequence being particular embodiments, in a sample obtained from the patient, wherein the sample is a full blood, blood serum or blood plasma sample, wherein determining the level of the biomarker comprises determining the protein concentration of the biomarker and comparing the level of the biomarker for atrial heart muscle tissue damage determined in the patient with the level of the biomarker for atrial heart muscle tissue damage observed in a control individual, wherein if the level of the biomarker for atrial heart muscle tissue damage determined in the patient is increased compared to the level of the biomarker for atrial heart muscle tissue damage in the control individual, said patient is diagnosed with a heart muscle tissue damage, for example atrial heart muscle tissue damage.

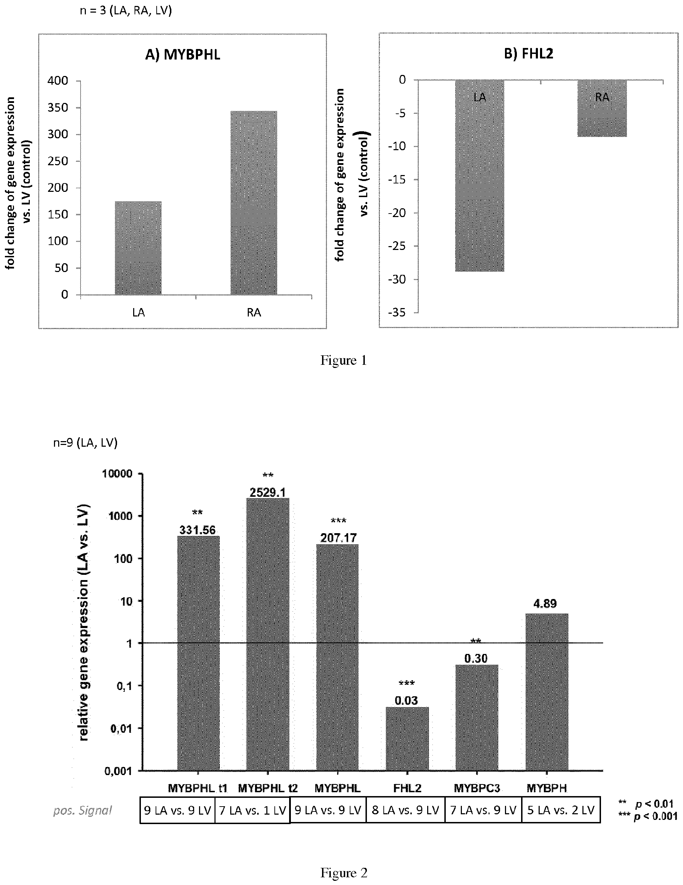

The inventors have identified Myosin-binding protein H-like (MBPHL= MYBPHL) as a biomarker for heart muscle tissue damage, for example as a specific biomarker for atrial heart muscle tissue damages (see example 1 and Figure 1). As shown in Figure 1 and Figure 2, MYBPHL gene is expressed in both the heart atrium and in the heart ventricle. However, the gene expression of MYBPHL in the atrium is clearly higher as in the ventricle, namely 150-fold higher in the left atrium and a 350-fold higher in the right atrium compared to the left ventricle. This means that, although an increase of the protein level of MYBPHL is detected, for example in the blood plasma of a patient, in the occurrence of a heart muscle tissue damage, the protein level of MYBPHL increases significantly more in the case the patient suffers from atrial heart muscle tissue damage as when the patient suffers from ventricular heart muscle tissue damage. Thus, MYBPHL can be used as specific biomarker for atrial heart muscle tissue damage.

According to the present invention, MYBPHL or an isoform thereof is used as a first biomarker for atrial heart muscle tissue damage. Said marker can be combined with any one or more of the other biomarkers for atrial heart muscle tissue damage, selected from the group consisting of MYBPH (Uniprot Database entry: Q13203), MYOT (Uniprot Database entry: Q9UBF9), ASNSD1 (Uniprot Database entry: Q9NWL6), CHFR (Uniprot Database entry: Q96EP1), NTN1 (Uniprot Database entry: 095631), RFC5 (Uniprot Database entry: P40937), POLI (Uniprot Database entry: Q9UNA4), COMP (Uniprot Database entry: P49747), POF1B (Uniprot Database entry: Q8WVV4), PLA2G2A (Uniprot Database entry: P14555), HDAC10 (Uniprot Database entry: Q969S8), ASPG (Uniprot Database entry: Q86U10), FMOD (Uniprot Database entry: Q06828), CA13 (Uniprot Database entry: Q8N1Q1), CACNA2D2 (Uniprot Database entry: Q9NY47), GNL3L (Uniprot Database entry: Q9NVN8), COL2A1 (Uniprot Database entry: P02458), PDLIM4 (Uniprot Database entry: P50479), LEPREL1 (Uniprot Database entry: Q8IVL5), OMD (Uniprot Database entry: Q99983), DGKZ (Uniprot Database entry: Q13574), NT5DC2 (Uniprot Database entry: Q9H857), ITLN1 (Uniprot Database entry: Q8WWA0), NTM (Uniprot Database entry: Q9P121), PRKG2 (Uniprot Database entry: Q13237), CHGB (Uniprot Database entry: P05060), FAM179A (Uniprot Database entry: Q6ZUX3), CKMT1A / CKMT1B (Uniprot Database entry: P12532), LTBP3 (Uniprot Database entry: Q9NS15), SGSM1 (Uniprot Database entry: Q2NKQ1), METTL7B (Uniprot Database entry: Q6UX53), LTBP2 (Uniprot Database entry: Q14767), OGDHL (Uniprot Database entry: Q9ULD0), PAM (Uniprot Database entry: P19021), SBK3 (Uniprot Database entry: P0C264), SFRP1 (Uniprot Database entry: Q8N474) and/or isoforms thereof. In various embodiments, it can be combined with MYBPH or an isoform thereof. Accordingly, in various embodiments of the invention, one or more, such as 1, 2, 3, 4, 5, 6, 7, 8, 9, 10 or more, such as 20, 30 or all 37 of the above-listed biomarkers for atrial heart muscle tissue damage can be used. The can be combined with any one of more of the further biomarkers for general heart muscle damage, disclosed above.

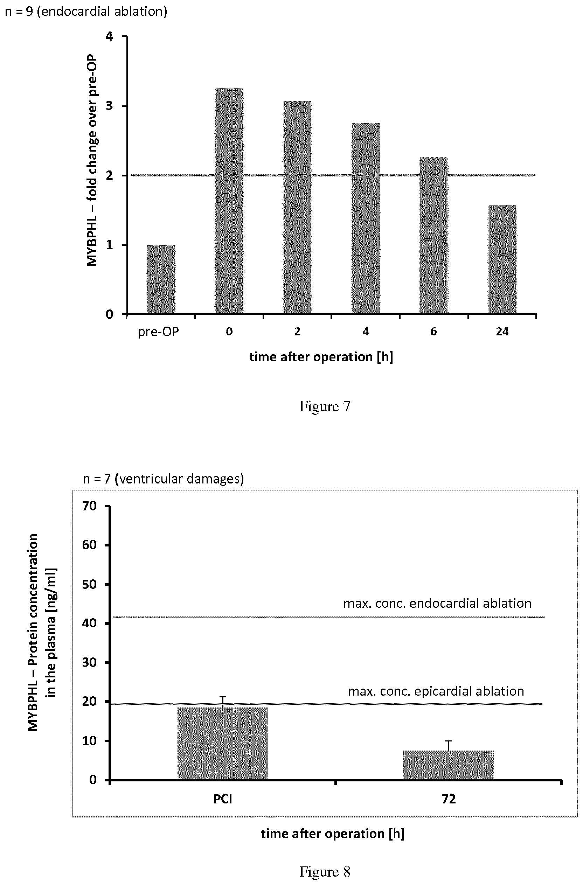

Figure 8 shows the results of the measurement of MYBPHL protein concentration in a blood plasma sample taken from a group of patients suffering from ST-elevation myocardial infarction, e.g. patients with a definitive damage in the ventricle, before and after a surgical intervention to treat the ST-elevation myocardial infarction. Large ST elevated myocardial infarctions leads to combined damages in the ventricle and the atrium. Figure 8 shows that the MYBPHL protein concentration, before the surgical intervention, is increased due to the ventricular damage and the atrial damage. 72 hours after the intervention to treat the ST-elevation myocardial infarction, the MYBPHL protein concentration is reduced. Thus, the results of the experiment showed in Figure 8 confirm the suitability of the biomarker MYBPHL as biomarker for heart muscle tissue damage.

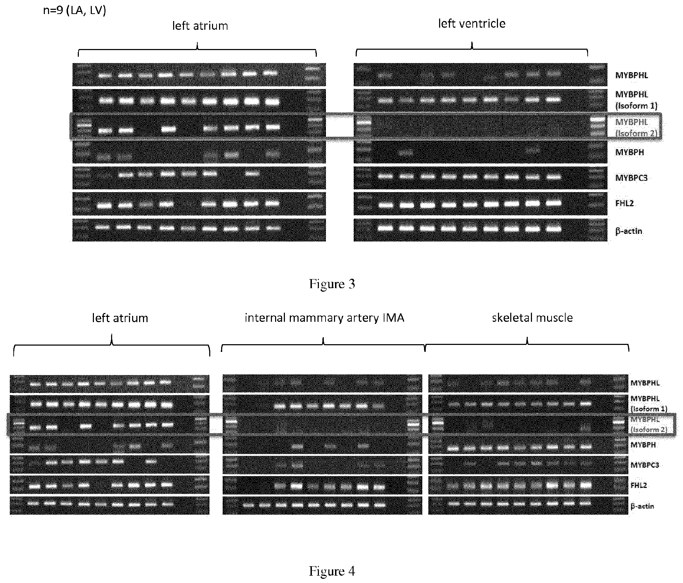

In addition, the inventors have identified Myosin-binding protein H (MBPH =MYBPH), and isoforms of MYBPHL, e.g. MYBPHL isoform 1 and MYBPHL isoform 2, as additional suitable biomarkers for the diagnosis of atrial heart muscle tissue damages. Figure 2 shows that, although MYBPHL isoform 1 gene is expressed in the 9 left studied atria as well as in the 9 left studied ventricles, MYBPHL isoform 1 gene expression is around 210-fold higher in the atrium as in the ventricle. MYBPH gene and MYBPHL isoform 2 gene are mostly expressed in the left atria (5 left atria versus 2 left ventricles for MYBPH and 7 left atria versus 1 left ventricle for MYBPHL isoform 2). Figure 3, which shows the separation of the amplification products of the biomarker MYBPHL isoform 2 from left atria and from left ventricles, confirms this observation. Hence the biomarkers MYBPH and MYBPHL isoform 2 are highly specific biomarkers for atrial heart muscle tissue damages. Figure 4 shows the separation of the amplification products of the biomarker MYBPHL isoform 2 in non-cardiac tissues. In Figure 4, MYBPHL isoform 2 was not observed in non-cardiac tissues like arterial vessels containing smooth muscle cells and skeletal muscle samples, confirming the specificity of MYBPLH isoform 2 for heart muscle, specifically for the left atrium, is shown. In contrast MYBPH showed an enriched expression in skeletal muscle.

The level of the biomarker for atrial heart muscle tissue damage observed in a control individual is the level of the biomarker determined in a normal healthy individual or in a control patient. A normal healthy individual is a person for whom no or insignificant heart problems have been observed. A control patient may be a patient suffering from a heart condition, for example about to undergo a surgery like a coronary artery bypass grafting or a valve surgery, and on which a MAZE procedure consisting of an ablation of the atrium, i.e. atrial heart muscle tissue damage, will be performed during the surgery. A control patient may also be a patient suffering from a heart defect and who had undergone an isolated aortic valve replacement by means of a heart-lung machine, since such a patient usually do not show alteration in the atrium. Furthermore, the control patient may be a patient who had undergone a catheter-based implantation of a heart valve without the use of a heart-lung machine.

An increased level of the biomarker in the patient means that its concentration is increased relative to the control individual. This term includes that, in the normal healthy individual, the biomarker is detectable at a concentration that remains stable or nearly stable over time while the biomarker concentration in a patient having heart muscle tissue damage varies and is higher than the biomarker concentration measured in the normal healthy individual. It is also possible to define a ratio, also called fold-change, between the biomarker level in the patient and the biomarker level in the control individual, wherein, when the ratio is in a range from 1.5 to 40, e.g. at least 2, 3, 4, 5, 6, 10, 12, 15, 20, 30, the level of the biomarker in the patient is defined as increased compared to the level in the control individual. In various embodiments, an "increased level" refers to an at least 1.5-fold increase, preferably an at least 2-fold increase, relative to the level observed in a healthy individual.

The level observed in a control/healthy individual may also be an average value calculated based on levels determined in a plurality of control/healthy individuals. The healthy/control individual level may also be the level of the patient before said patient underwent therapy or surgery or experienced an event that caused heart muscle damage. The terms "healthy individual" and "control individual" are used interchangeably herein.

In certain embodiments of the method described herein, the method comprises evaluating the extent of heart muscle tissue damage in the patient by determining the ratio (also called fold-change) between the level of the biomarker for atrial heart muscle tissue damage in the sample obtained from the patient and the level of the biomarker for atrial heart muscle tissue damage in a healthy/control individual.

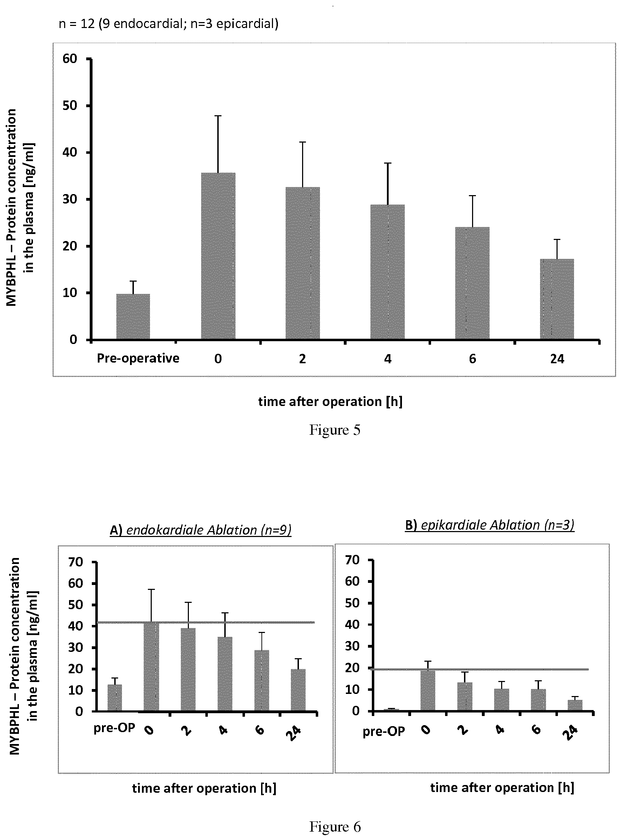

Figure 5 shows the evolution of the MYBPHL protein concentration in blood samples of patients undergoing MAZE procedure, an ablation of the atrium, i.e. atrial heart muscle tissue damage. As shown in Figure 5, the MYBPHL protein concentration is the highest MYBPHL protein concentration after the atrium ablation, namely at the time point of the arrival of the patient at the intensive care unit. The MYBPHL protein concentration decreases over time, for example within 24 hours after the ablation, as no further atrial heart muscle tissue damage is occurring. Also Figure 6 shows the evolution of the MYBPHL protein concentration in blood samples of patients undergoing endocardial ablation or epicardial ablation. Endocardial ablation is a more aggressive form of ablation compared to epicardial ablation. As shown in Figure 6, the MYBPHL protein concentration is higher in patients with endocardial ablation as in patients with epicardial ablation. Hence, as shown in the experiments illustrated in Figure 5 and Figure 6, the MYBPHL protein concentration and therefore its fold-change is proportional to the extent of the atrial heart muscle tissue damage. In other words, the bigger the heart muscle tissue damage is, the higher the MYBPHL level is. Furthermore, the biomarker MYBPHL has the advantage to be a sensitive biomarker, since the MYBPHL level is still detectable 24 hours after atrial ablation, whereas the known biomarker Myoglobin is not detected anymore in a sample of the patient after 24 hours.

In various embodiments of the method described herein, the method further comprises, in the sample of the patient, determining the level of a further biomarker for heart muscle tissue damage, wherein the method is suitable to distinguish between atrial muscle tissue damage and ventricular muscle tissue damage.

The differentiation between atrial muscle tissue damage and ventricular muscle tissue damage can be achieved by determining the level of a further biomarker for heart muscle tissue damage in the sample of the patient. The method may further comprise comparing the level of the further biomarker with the level of the biomarker specific for atrial heart muscle tissue damage, and/or comparing the level of the further biomarker in the sample of the patient with the level of the further biomarker in a sample obtained from a control individual to obtain the fold-change. The further biomarker for heart muscle tissue damage can be a general biomarker for heart muscle tissue damage selected from the group consisting of CK-MB or Troponin. Also, the further biomarker for heart muscle tissue damage can be a biomarker specific for ventricular heart muscle tissue damage. As shown in Figure 2, FHL-2 is a biomarker specific for ventricular heart muscle tissue damage, since expression levels of the FHL-2 gene are almost 50 times higher in the left ventricle compared to the left atrium.

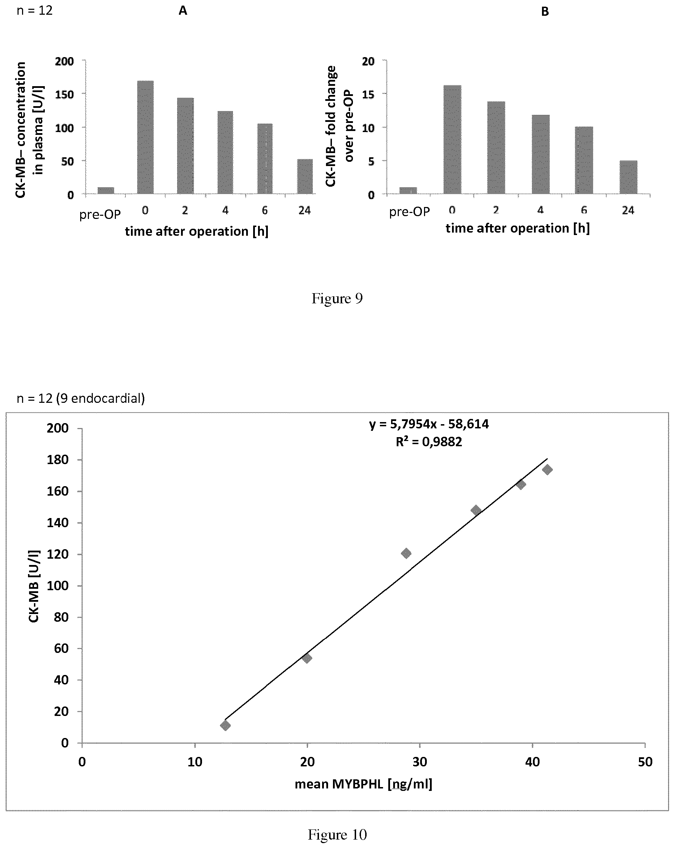

When the further biomarker for heart muscle tissue damage is a general biomarker for heart muscle tissue damage, preferably selected from the group consisting of CK-MB or Troponin, the ratio between the protein concentration of the general biomarker in the sample of the patient and the protein concentration of the biomarker for atrial heart muscle tissue damage may be calculated. Alternatively or additionally, the level of the biomarker for atrial heart muscle tissue damage and the level of the general biomarker for heart muscle tissue damage can be determined at periodical measurement time points over a defined period of time, for example every two hours, for example over 24 hours or 72 hours. If the level of the biomarker for atrial heart muscle tissue damage and the level of the general biomarker for heart muscle tissue damage both decrease over the defined period of time, for example proportionally to each other, the patient is diagnosed with an atrial heart muscle tissue damage (see example 5, Figure 9 and Figure 10). If the level of the biomarker for atrial heart muscle tissue damage decreases when the level of the general biomarker for heart muscle tissue damage remains stable or increases, the patient is diagnosed with ventricular muscle tissue damage.

When the further biomarker for a heart muscle tissue damage is a biomarker specific for ventricular heart muscle tissue damage, preferably FHL-2, the ratio between the fold-change of MYBPHL and the fold-change of FHL-2 is calculated, for example at periodical measurement time points over a defined period of time, for example every two hours, for example over 24 hours or 72 hours. When the ratio between the fold-change of MYBPHL and the fold-change of FHL-2 is above 1,5, for example in a range from 1,5 to 4, the patient is diagnosed with atrial heart muscle tissue damage (see example 6, Figure 10).

Also, the differentiation between atrial muscle tissue damage and ventricular muscle tissue damage can be achieved by measuring the level of the biomarkers very specific for atrial heart muscle tissue damages selected from the group consisting of MYBPH or MYBPHLt2 in a sample of the patient and comparing these levels with the level of these very specific biomarkers in a control individual. When the ratio between the level of the very specific biomarkers in the sample of the patient and the level of the very specific biomarkers in the sample of a control individual is in the range from 1,5 to 40, e.g. 2, 3, 4, 5, 6, 10, 12, 15, 20, 30, the patient is diagnosed with atrial heart muscle tissue damage.

According to the present invention, the sample obtained from the patient is full blood, blood plasma or blood serum.

In some embodiments the patient is a mammal, preferably a human.

Generally, the term "mammal", as used herein, comprises human, monkeys, pigs, cows, cats, dogs, guinea pigs, rabbits, mice, sheep, goats and horses.

For the detection of the markers of the present invention specific binding partners may be employed. In some embodiments, the specific binding partners are useful to detect the presence of a marker in a sample, wherein the marker is a protein. The marker and its binding partner represent a binding pair of molecules, which interact with each other through any of a variety of molecular forces including, for example, ionic, covalent, hydrophobic, van der Waals, and hydrogen bonding. Preferably, this binding is specific. "Specific binding" means that the members of a binding pair bind preferentially to each other, i.e. usually with a significant higher affinity than to non-specific binding partners. The binding affinity for specific binding partners is thus usually at least 10-fold, preferably at least 100-fold higher than that for non-specific binding partners.

Exemplary binding partners for the markers of the invention are selected from the group consisting of antibodies, antibody fragments and variants, molecules with antibody-like properties, such as lipocalin muteins or Spiegelmers or aptamers. Antibody fragments and variants include Fv fragments, linear single chain antibodies and the like all of which are known to those skilled in the art. Particularly preferred are antibodies.

According to the present invention, determining the level of a biomarker specific for atrial heart muscle tissue damage comprises determining the amount of protein of the biomarker present in a sample.

In various embodiments, at least one or more markers are determined at mRNA level and at least one or more markers are determined at protein level.

If a marker is determined on mRNA level, the mRNA may be the mRNA transcript, a 5'- and/or 3'-truncated mRNA or spliced mRNA forms.

If a marker is determined on protein level, the protein may be the full length protein or a fragment thereof. The protein fragment may be a truncated protein, i.e. lack one or more amino acids, for example, at the N-terminus or C-terminus or both. This may be due to post-translational processing or due to the action of proteases present in the cell or the sample. The markers determined in the methods of the invention thus also include naturally occurring fragments, preferably immunogenic fragments. Also, the protein may be posttranslationally modified, e.g., phosphorylated, hydroxylated, glycosylated, N-glycosylated, O-glycosylated, ubiquitinylated, acetyated, methylated, prenylated or sulphated. It is understood that if such fragments are to be determined, the sequence identities given relate to the entire sequence of the fragment relative to the entire length of the corresponding part of the reference sequence.

According to the present invention, the method features the determination of the markers on protein level, i.e. MYBPHL and its respective isoforms in a sample obtained from the patient, wherein the sample is a full blood, blood serum or blood plasma sample. Such methods may preferably be performed on blood or serum samples obtained from a patient.

In certain embodiments of the methods of the invention, the levels of at least 2, 3, 4, 5, 6, 7, 8, 9, 10, 20, 50 or 100 or more biomarkers are determined.

In the method of the present invention, determining the level of a biomarker for atrial heart muscle tissue damage comprises determining the protein level of the biomarker for atrial heart muscle tissue damage in a sample.

Accordingly, in some embodiments the methods involve the determination of the level of the biomarkers on protein level only.

The methods detailed above, wherein the level of at least one or more markers is determined on protein level comprise in some embodiments the determination of the protein level by an immunoassay, mass spectrometry, chromatography, Western Blot, or gel electrophoresis.

In some embodiments, the immunoassay may be, but is not limited to an Enzyme-linked Immunosorbent Assay (ELISA), Western blot, agglutination test, biotin/avidin type assay, radioimmunoassay, immunoelectrophoresis and immunoprecipitation. The reactions generally include revealing labels such as fluorescent, chemiluminescent, radioactive, enzymatic labels or dye molecules, or other methods for detecting the formation of a complex between the antigen and the antibody or antibodies reacted therewith. These and further immunoassays are well known in the art (David Wild (Ed.): The Immunoassay Handbook. 3rd ed. Elsevier Science Publishing Company, Amsterdam 2005).

The aforementioned assays may involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with an antibody against the protein to be tested. A biological sample containing or suspected of containing the marker is then added to the coated wells. After a period of incubation sufficient to allow the formation of antibody-antigen complexes, the plate(s) can be washed to remove unbound moieties and a detectably labeled secondary binding molecule added. The secondary binding molecule is allowed to react with any captured sample marker protein, the plate washed and the presence of the secondary binding molecule detected using methods well known in the art.

In certain embodiments of the above detailed methods, if the determination is via mass spectrometry, the mass spectrometry may be selected from the group comprising MS measurements using El, CI, ESI, APLI, APPI and APCI.

The biomarker determination on protein level employing chromatography may be selected from the group comprising liquid chromatography, HPLC, FPLC, Smart chromatography, gel chromatography, size exclusion chromatography, reverse phase chromatography and ion-exchange chromatography (Introduction to Modern Liquid Chromatography, Lloyd R. Snyder, 5 Wiley, 2009).

In various embodiments, if the biomarker is detected via gel electrophoresis, the gel electrophoresis may be selected from the group, but not limited to agarose gel electrophoresis, sodium dodecyl sulfate poly acryl amide gel electrophoresis (SDS-PAGE), 2D-gel electrophoresis, native gel electrophoresis and quantitative preparative native continuous polyacrylamide gel 10 electrophoresis (QPNC-PAGE).

Of course, in certain embodiments of the methods of the present invention at least two determination methods may be coupled to each other in a subsequent manner. In a variant, gel electrophoresis may be followed by mass spectroscopic analysis. Alternatively, gel electrophoresis may be followed by Western Blot, chromatography may be followed by 15 mass spectroscopic analysis, chromatography may be followed by an immunoassay, e.g. ELISA.

In further embodiments of the methods of the present invention, determining the level of the biomarker for atrial heart muscle tissue damage also comprises determining the mRNA level of the biomarker for atrial heart muscle tissue damage in a sample, i.e., the level of the one or more biomarkers is determined at protein and mRNA level.

Where in the methods detailed above a biomarker is determined on mRNA level, the RNA level may be determined by PCR, gel electrophoresis and/or Northern Blot.

In case the biomarker level is determined on the RNA level, the detection reagent may be a nucleic acid molecule, such as an oligonucleotide. The oligonucleotide may be a nucleic acid probe that may be labeled to allow detection or may be an oligonucleotide primer that allows amplification of the target molecule.

### Examples

### Example 1: Increased MYBPHL, MYBPH, MYBPHL isoform 1 and MYBPHL isoform 2 gene expression in atrial heart muscle tissues (Reference Example)

A study of the human heart proteome was performed, for example according to "Region and cell-type resolved quantitative proteomic map of the human heart" Doll S, Dreßen M, Geyer PE, Itzhak DN, Braun C, Doppler SA, Meier F, Deutsch MA, Lahm H, Lange R, Krane M, Mann M. Nat Commun. 2017 Nov 13;8(1):1469. doi: 10.1038/s41467-017-01747-2. PMID: 29133944. The expression of various biomarker in heart tissue samples obtained from patients who underwent heart surgery by means of biopsy has been validated by qPCR. In the study tissue samples were taken out from the following areas (wherein the number of tissue sample was n=3): left atrium (LA), right atrium (RA), left ventricle (LV) and right ventricle (RV).

Figure 1 shows the results of gene expression analysis of the biomarker MYBPHL and the biomarker FHL2, wherein the gene expression analysis has been performed by qPCR. Figure 1A shows a highly significant differential in the expression of MYBPHL in the left atrium and in the right atrium compared to the left ventricle. Thereby the gene expression of MYBPHL in the right atrium is 300 times higher than in the left ventricle. Figure 1B shows a significant differential in the expression of FHL2 in the left ventricle compared to the left atrium and the right atrium, wherein the gene expression of FHL 2 in the left ventricle is 28 times higher than in the left atrium.

Figure 2 shows the results of gene expression analysis of the biomarker MYBPHL, FHL-2, MYBPC3 and MYBPH as well as the MYBPHL isoforms MYBPHL isoform 1 (MYBPHL t1) and MYBPHL isoform 2 (MYBPHL t2) in tissues taken from 9 left ventricle muscle tissues and 9 left atrium muscle tissues obtained by means of a biopsy in a patient, wherein gene expression analysis has been performed by qPCR. Thereby gene expressions of MYBPHL, MYBPH and the two MYBPHL isoforms MYBPHL isoform 1 and MYBPHL isoform 2 are higher in the left atrium, wherein the differential expression of MYBPH is clearly lower than the one of MYBPHL (almost 42 times lower). FHL-2 is a gene, which is expressed at a higher level in the left ventricle. Furthermore MYBPHL isoform 2 shows a high specificity for the left atrium since MYBPHL isoform 2 is expressed only in 1 left ventricle sample out of 9 left ventricle samples, whereas MYBPHL isoform 2 is expressed in 7 left atrium samples out of 9 left atrium samples.

Figure 3 shows the results of gene expression analysis of the biomarker MYBPHL isoform 2, wherein gene expression analysis has been shown by gel electrophoresis. Thereby the high specificity of MYBPHL isoform 2 for the left atrium is confirmed, wherein MYBPHL isoform 2 has been detected in none of left ventricle samples, whereas MYBPHL isoform 2 has been detected in 7 left atrium samples out of 9 left atrium samples.

Figure 4 shows the expression level of MYBPHL isoform 2 in the left atrium as well as in non-cardiac tissues. Figure 4 shows that MYBPHL isoform 2 has been detected in none of the arterial vessels containing smooth muscle cells and skeletal muscle samples, whereas MYBPHL isoform 2 has been detected in the left atrium, confirming thereby the high cardiac specificity of MYBPHL isoform 2.

### Example 2: Detection of the biomarker MYBPHL in blood plasma

A study was performed to determine if the biomarker MYBPHL could be detected in blood plasma. A patient group who has undergone an ablation (modified MAZE procedure) for the treatment of atrial fibrillation besides heart surgery like coronary artery bypass grafting or valve surgery has been studied. In the MAZE procedure, focused and intended specific heart muscle damage has been performed either in endocardial heart muscle tissue or in epicardial heart muscle tissue of the patient. Over a time period of 24 hours at the following measurement time points, a blood sample has been taken out from the patient:

| | | |
|---|---|---|
| 1. | Pre-OP | before the beginning of the heart surgery |
| 2. | 0h | arrival of the patient in the intensive care unit |
| 3. | 2h | 2 hours after arrival of the patient in the intensive care unit |
| 4. | 4h | 4 hours after arrival of the patient in the intensive care unit |
| 5. | 6h | 6 hours after arrival of the patient in the intensive care unit |
| 6. | 24h | 24 hours after arrival of the patient in the intensive care unit |

Figure 5 shows the results of the measurement of MYBPHL protein concentration in the blood plasma samples obtained from the patient group of 12 patients (n = 12), who has undergone endocardial and epicardial ablation. The concentration of MYBPHL in the plasma sample increased clearly and significantly already at the measurement time point 2. At the measurement time point 2, the maximum of MYBPHL plasma concentration is reached. Over the time, the MYBPHL plasma concentration decreased gradually, wherein the MYBPHL plasma concentration at measurement time point 6, namely 24 hours after the arrival of the patient in the intensive care unit, still not have reached the preoperative concentration level. The data show that MYBPHL compared to Myoglobin, an unspecific biomarker know in the art, allows the detection in blood plasma of the occurrence of heart muscle damage even after more than 24 hours after its occurrence.

### Example 3: Damage extent dependence with the concentration of the biomarker MYBPHL in blood plasma

Figure 6 shows the results of the measurement of MYBPHL protein concentration in a blood plasma sample over a defined period of time and at measurement time points as described in Example 2 in patients having undergone either endocardial ablation (A) or epicardial ablation (B). In this study a group of 9 patients (n = 9) underwent endocardial ablation (A) and a group of 3 patients (n = 3) underwent epicardial ablation (B). Endocardial ablation is a more aggressive form of ablation as epicardial one and leads to stronger heart muscle damage. The postoperative values show for both groups of patients with endocardial ablation and epicardial ablation a significant increase of MYBPHL protein concentration in the blood plasma, which decreases over time. However, for patients who received epicardial ablation, the MYBPHL protein concentration in the blood plasma never reached a higher level than the MYBPHL protein concentration at all postoperative measurement times in the blood plasma of patients who received endocardial ablation. This shows the sensitivity and the correlation between the MYBPHL protein concentration and the present atrial heart muscle damage, since the MYBPHL protein concentration is higher by patients having received the more aggressive endocardial ablation.

Figure 7 shows the fold-change in the MYBPHL protein concentration in a blood plasma sample according to Figure 6. For the case of endocardial ablation, the preoperative MYBPHL plasma concentration has been arbitrary set at 1. In the case of endocardial ablation, a maximum of 3-fold MYBPHL concentration increase has been measured. The maximum corresponds to the measurement time point of the patient's arrival in the intensive care unit. On the other hand, in the case of epicardial ablation, a maximum of 20 fold MYBPHL concentration increase has been measured, wherein the intensity of the atrial heart muscle damage is lower, as described in Fig.5.

### Example 4: MYBPHL plasma concentration in patients suffering from an acute ST-elevation myocardial infarction

The MYBPHL protein concentration was measured in a patient group that presented acute ST-elevation myocardial infarction. Figure 8 shows the results of the measurement of MYBPHL protein concentration in blood plasma samples. Blood samples were taken at two measurement time points, one before intervention and one 72 hours after the intervention. The intervention was performed by means of a heart catheter. Due to the pathology of acute ST-elevation myocardial infarction, the patients of this group suffered from a definitive ventricle damage along with a atrial cardiac damage as shown in Figure 8, where the highest MYBPHL plasma concentration is found at the measurement time point 1, e.g. before the intervention. This MYBPHL plasma concentration is similar to the MYBPHL plasma concentration found in patients treated with an epicardial MAZE. 72 hours after the intervention, the MYBPHL plasma concentration decreased to values similar to the ones obtained in example 3 before patients were treated with MAZE (figure 5 and figure 6a) or in healthy subjects. The data of this study show that the MYBPHL plasma concentration highly increases in the case of an acute heart muscle damage and decreases over time to reach normal values when the pathology is not anymore present.

### Example 5: Proportionality between the protein concentration of the specific biomarker MYBPHL and the protein concentration of the known unspecific biomarker CK-MB in the case of atrial heart muscle tissue damage

Figure 9A shows the results of the measurement of CK-MB protein concentration in blood serum samples obtained from the same patient group as in example 2. Figure 9B shows the fold-change in the CK-MB protein concentration in the blood serum sample according to Figure 9A. Compared to the MYBPHL, the CK-MB protein concentration reached its maximum value also at the measurement time point 2. At this measurement time point 2, the CK-MB protein concentration is 15 times higher as the CK-MB protein concentration before the ablation.

Figure 10 shows a correlation between the MYBPHL concentration values in a blood plasma sample of 12 patients who underwent an ablation and the CK-MB concentration values in a blood serum sample of the same patients at the same measurement times. CK-MB, although unspecific for atrial muscle damage or for ventricular muscle damage, is an established biomarker for the detection of heart muscle damage, wherein its concentration is known as being correlated with the extent of the heart muscle damage. As shown in Figure 10 a 1-to-1 correlation could be proved between MYBPHL concentration values and CK-MB concentration values.

### Example 6: Differentiation between atrial heart muscle tissue damage and ventricular heart muscle tissue damage using the fold-change of the biomarker MYBPHL and the fold-change of the biomarker FHL-2

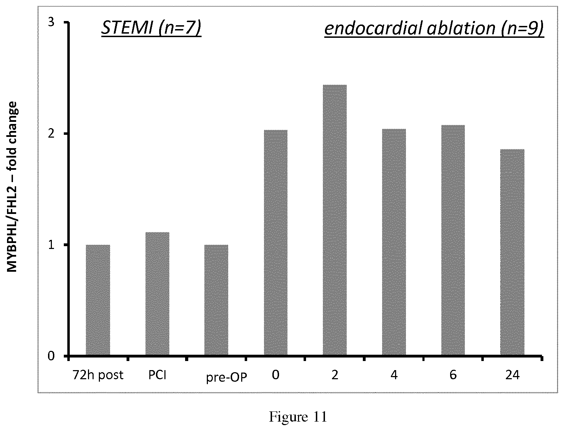

Figure 11 shows a representation of the ratio between the fold-change of MYBPHL and the fold-change of FHL-2. On the two left columns of the representation, the results for patients suffering from ST-elevation myocardial infarction are shown, while on the six right columns of the representation, the results for patients suffering from atrial fibrillation and undergoing an ablation are shown. The ratio between the fold-change of MYBPHL and the fold-change of FHL-2 increases up to 2 for patients having undergone atrial ablation. On the contrary the ratio between the fold-change of MYBPHL and the fold-change of FHL-2 remains stable at about 1 before and after the intervention for patients suffering from ST-elevation myocardial infarction, e.g. ventricular heart muscle tissue damage.

## Claims

1. A method of diagnosing heart muscle tissue damage, preferably atrial heart muscle tissue damage, in a patient, wherein the method comprises:
(a) determining the level of a biomarker for atrial heart muscle tissue damage selected from the group consisting of MYBPHL and isoforms thereof in a sample obtained from the patient, wherein the sample is a full blood, blood serum or blood plasma sample, wherein determining the level of the biomarker comprises determining the protein concentration of the biomarker; and
(b) comparing the level determined in step a) with the level of the biomarker for atrial heart muscle tissue damage observed in a control individual, wherein if the level determined in step a) is increased compared to the level of the biomarker for atrial heart muscle tissue damage in the control individual, said patient is diagnosed with a heart muscle tissue damage.

2. The method of claim 1, wherein the isoforms of MYBPHL are selected from the group consisting of MYBPHL isoform 1 and MYBPHL isoform 2.

3. The method of claim 2, wherein MYBPHL isoform 1 and MYBPHL isoform 2 have the amino acid sequence as set forth in SEQ ID NO:3 or 6, or functional homologs thereof having at least 85 % sequence identity thereto over the entire length.

4. The method of any one of claims 1 to 3, wherein in step (a) the level of one or more further biomarkers for atrial heart muscle damage are determined, said biomarkers being selected from MYBPH, MYOT, ASNSD1, CHFR, NTN1, RFC5, POLI, COMP, POF1B, PLA2G2A, HDAC10, ASPG, FMOD, CA13, CACNA2D2, GNL3L, COL2A1, PDLIM4, LEPREL1, OMD, DGKZ, NT5DC2, ITLN1, NTM, PRKG2, CHGB, FAM179A, CKMT1A, CKMT1B, LTBP3, SGSM1, METTL7B, LTBP2, OGDHL, PAM, SBK3, SFRP1 and isoforms thereof and in step (b) the levels of said biomarkers determined in step a) are compared with the levels of the respective marker(s) in a control individual.

5. The method of any one of claims 1 to 4, wherein the method further includes determining the extent of the heart muscle tissue damage in said patient, the extent of heart muscle damage being evaluated by determining the ratio between the level of the biomarker for atrial heart muscle tissue damage in the sample obtained from said patient and the level of the biomarker for atrial heart muscle tissue damage in a control individual.

6. The method of any one of claims 1 to 5, wherein the method further comprises:
(c) in the sample of said patient, determining the level of a further biomarker for a heart muscle tissue damage,
wherein the method is suitable to differentiate between atrial muscle tissue damage and ventricular muscle tissue damage.

7. The method of claim 6, wherein the further biomarker is a biomarker specific for ventricular heart muscle tissue damage, preferably FHL-2, SMYD2, FASTKD1, PRR33, SORBS2, CRISPLD1, NPHP4, ANKRD2, TMEM159, ATP7A, C12ORF73, NAV1, ATP5B, HSPB7, TMEM88, WDR62, ACTN3, TRIM72, EGFLAM, LRRC39, DPYSL4, PFKFB2, ABCB6, METTL2B, METTL2A, CARNS1, SPTBN1or isoforms thereof.

8. The method of any one of claims 1 to 6, wherein the further biomarker is a general biomarker for heart muscle tissue damage, preferably selected from the group consisting of CK-MB or Troponin,

9. The method of any one of claims 1 to 8, wherein the sample is a blood serum or a blood plasma.

10. The method of any one of claims 1 to 9, wherein the patient is a human.

11. The method of any one of claims 1 to 10, wherein the protein concentration is determined by an immunoassay, ELISA, mass spectrometry, chromatography, Western Blot, or gel electrophoresis.

## Patentansprüche

1. Ein Verfahren zur Diagnose von Herzmuskelgewebeschäden, bevorzugt von Vorhof-Herzmuskelgewebeschäden, bei einem Patienten, wobei das Verfahren umfasst:
(a) Bestimmen des Niveaus eines Biomarkers für Vorhof-Herzmuskelgewebeschäden ausgewählt aus der Gruppe bestehend aus MYBPHL und Isoformen davon in einer vom Patienten erhaltenen Probe, wobei die Probe eine Vollblut-, Blutserum- oder Blutplasma-Probe ist, wobei das Bestimmen des Niveaus des Biomarkers das Bestimmen der Proteinkonzentration des Biomarkers umfasst; und
(b) Vergleichen des in Schritt a) bestimmten Niveaus mit dem Niveau des Biomarkers für Vorhof-Herzmuskelgewebeschäden, das in einem Kontrollindividuum beobachtet wurde, wobei, wenn das in Schritt a) bestimmte Niveau, verglichen mit dem Niveau des Biomarkers für Vorhof-Herzmuskelgewebeschäden des Kontrollindividuums erhöht ist, bei dem Patienten eine Schädigung des Herzmuskelgewebes diagnostiziert wird.

2. Das Verfahren gemäß Anspruch 1, wobei die Isoformen von MYBPHL ausgewählt sind aus der Gruppe bestehend aus MYBPHL-Isoform 1 und MYBPHL-Isoform 2.

3. Das Verfahren gemäß Anspruch 2, wobei MYBPHL-Isoform 1 und MYBPHL-Isoform 2 die in SEQ ID NO:3 oder 6 dargestellte Aminosäuresequenz aufweisen, oder funktionelle Homologe davon, die mindestens 85 % Sequenzidentität damit über die gesamte Länge aufweisen.

4. Das Verfahren gemäß einem der Ansprüche 1 bis 3, wobei in Schritt (a) das Niveau eines oder mehrerer weiterer Biomarker für Vorhof-Herzmuskelgewebeschäden bestimmt wird, wobei die Biomarker ausgewählt sind aus MYBPH, MYOT, ASNSD1, CHFR, NTN1, RFC5, POLI, COMP, POF1B, PLA2G2A, HDAC10, ASPG, FMOD, CA13, CACNA2D2, GNL3L, COL2A1, PDLIM4, LEPREL1, OMD, DGKZ, NT5DC2, ITLN1, NTM, PRKG2, CHGB, FAM179A, CKMT1A, CKMT1B, LTBP3, SGSM1, METTL7B, LTBP2, OGDHL, PAM, SBK3, SFRP1 und Isoformen davon und in Schritt (b) die Niveaus der in Schritt (a) bestimmten Biomarker mit den Niveaus des/der jeweiligen Marker(s) in einem Kontrollindividuum verglichen werden.

5. Das Verfahren gemäß einem der Ansprüche 1 bis 4, wobei das Verfahren ferner das Bestimmen des Ausmaßes der Herzmuskelgewebeschäden bei dem Patienten einschließt, wobei das Ausmaß der Herzmuskelschäden durch das Bestimmen des Verhältnisses zwischen dem Niveau des Biomarkers für Vorhof-Herzmuskelgewebeschäden in der vom Patienten erhaltenen Probe und dem Niveau des Biomarkers für Vorhof-Herzmuskelgewebeschäden in einem Kontrollindividuum evaluiert werden.

6. Das Verfahren gemäß einem der Ansprüche 1 bis 5, wobei das Verfahren ferner umfasst:
(c) Bestimmen des Niveaus eines weiteren Biomarkers für Herzmuskelgewebeschäden in der Probe des Patienten,
wobei das Verfahren geeignet ist zwischen Vorhofmuskelgewebeschäden und ventrikulären Muskelgewebeschäden zu unterscheiden.

7. Das Verfahren gemäß Anspruch 6, wobei der weitere Biomarker ein Biomarker spezifisch für ventrikuläre Herzmuskelgewebeschäden ist, bevorzugt FHL-2, SMYD2, FASTKD1, PRR33, SORBS2, CRISPLD1, NPHP4, ANKRD2, TMEM159, ATP7A, C12ORF73, NAV1, ATP5B. HSPB7, TMEM88, WDR62, ACTN3, TRIM72, EGFLAM, LRRC39, DPYSL4, PFKFB2, ABCB6, METTL2B, METTL2A, CARNS1, SPTBN1 oder Isoformen davon.

8. Das Verfahren gemäß einem der Ansprüche 1 bis 6, wobei der weitere Biomarker ein allgemeiner Biomarker für Herzmuskelgewebeschäden ist, bevorzugt ausgewählt aus der Gruppe bestehend aus CK-MB oder Troponin.

9. Das Verfahren gemäß einem der Ansprüche 1 bis 8, wobei die Probe eine Blutserum- oder eine Blutplasma-Probe ist.

10. Das Verfahren gemäß einem der Ansprüche 1 bis 9, wobei der Patient ein Mensch ist.

11. Das Verfahren gemäß einem der Ansprüche 1 bis 10, wobei die Proteinkonzentration durch Immunoassay, ELISA, Massenspektrometrie, Chromatographie, Western Blot oder Gelelektrophorese bestimmt wird.

## Revendications

1. Procédé de diagnostic de l'endommagement du tissu du muscle cardiaque, de préférence de l'endommagement du tissu du muscle cardiaque atrial, chez un patient, sachant que le procédé comprend :
(a) la détermination du niveau d'un biomarqueur pour l'endommagement du tissu du muscle cardiaque atrial sélectionné dans le groupe constitué par MYBPHL et des isoformes de celui-ci dans un échantillon obtenu à partir du patient, sachant que l'échantillon est un échantillon de sang entier, de sérum sanguin ou de plasma sanguin, sachant que la détermination du niveau du biomarqueur comprend la détermination de la concentration en protéine du biomarqueur ; et
(b) la comparaison du niveau déterminé à l'étape a) avec le niveau du biomarqueur pour l'endommagement du tissu du muscle cardiaque atrial observé chez un individu témoin, sachant que si le niveau déterminé à l'étape a) est augmenté comparé au niveau du biomarqueur pour l'endommagement du tissu du muscle cardiaque atrial chez l'individu témoin, ledit patient est diagnostiqué avec un endommagement du tissu du muscle cardiaque.

2. Le procédé de la revendication 1, sachant que les isoformes de MYBPHL sont sélectionnées dans le groupe constitué par l'isoforme 1 de MYBPHL et l'isoforme 2 de MYBPHL.

3. Le procédé de la revendication 2, sachant que l'isoforme 1 de MYBPHL et l'isoforme 2 de MYBPHL présentent la séquence d'acides aminés telle qu'exposée dans SEQ ID N° 3 ou 6, ou des homologues fonctionnels de celle-ci ayant au moins 85 % d'identité de séquence avec celle-ci sur toute la longueur.

4. Le procédé de l'une quelconque des revendications 1 à 3, sachant qu'à l'étape (a) le niveau d'un ou de plusieurs biomarqueurs supplémentaires pour l'endommagement du muscle cardiaque atrial est déterminé, lesdits biomarqueurs étant sélectionnés parmi MYBPH, MYOT, ASNSD1, CHFR, NTN1, RFC5, POLI, COMP, POF1B, PLA2G2A, HDAC10, ASPG, FMOD, CA13, CACNA2D2, GNL3L, COL2A1, PDLIM4, LEPREL1, OMD, DGKZ, NT5DC2, ITLN1, NTM, PRKG2, CHGB, FAM179A, CKMT1A, CKMT1B, LTBP3, SGSM1, METTL7B, LTBP2, OGDHL, PAM, SBK3, SFRP1 et des isoformes de ceux-ci et à l'étape (b) les niveaux desdits biomarqueurs déterminés à l'étape a) sont comparés avec les niveaux du (des) marqueur(s) respectif(s) chez un individu témoin.

5. Le procédé de l'une quelconque des revendications 1 à 4, sachant que le procédé inclut en outre la détermination de l'étendue de l'endommagement du tissu du muscle cardiaque chez ledit patient, l'étendue de l'endommagement du muscle cardiaque étant évaluée en déterminant le rapport entre le niveau du biomarqueur pour l'endommagement du tissu du muscle cardiaque atrial dans l'échantillon obtenu à partir dudit patient et le niveau du biomarqueur pour l'endommagement du tissu du muscle cardiaque atrial chez un individu témoin.

6. Le procédé de l'une quelconque des revendications 1 à 5, sachant que le procédé comprend en outre :
(c) dans l'échantillon dudit patient, la détermination du niveau d'un biomarqueur supplémentaire pour un endommagement du tissu du muscle cardiaque,
sachant que le procédé est apte à différencier entre un endommagement du tissu du muscle atrial et un endommagement du tissu du muscle ventriculaire.

7. Le procédé de la revendication 6, sachant que le biomarqueur supplémentaire est un biomarqueur spécifique pour l'endommagement du tissu du muscle cardiaque ventriculaire, de préférence FHL-2, SMYD2, FASTKD1, PRR33, SORBS2, CRISPLD1, NPHP4, ANKRD2, TMEM159, ATP7A, C12ORF73, NAV1, ATP5B, HSPB7, TMEM88, WDR62, ACTN3, TRIM72, EGFLAM, LRRC39, DPYSL4, PFKFB2, ABCB6, METTL2B, METTL2A, CARNS1, SPTBN1 ou des isoformes de ceux-ci.

8. Le procédé de l'une quelconque des revendications 1 à 6, sachant que le biomarqueur supplémentaire est un biomarqueur général pour l'endommagement du tissu du muscle cardiaque, de préférence sélectionné dans le groupe constitué par CK-MB ou la troponine.

9. Le procédé de l'une quelconque des revendications 1 à 8, sachant que l'échantillon est un sérum sanguin ou un plasma sanguin.

10. Le procédé de l'une quelconque des revendications 1 à 9, sachant que le patient est un humain.

11. Le procédé de l'une quelconque des revendications 1 à 10, sachant que la concentration en protéine est déterminée par un immunoessai, ELISA, spectrométrie de masse, chromatographie, Western Blot, ou électrophorèse en gel.
